# EUROPEAN PATENT APPLICATION

(11) **EP 2 025 237 A1**
(43) Date of publication of application: **18.02.2009**
(21) Application number: 07114357.2
(22) Date of filing: 15.08.2007
(51) Int. Cl.: A23D 9/013, C11B 5/00

(54) **Lecithin and LC-PUFA**

(71) Applicant: Nestec S.A., 1800 Vevey (CH)
(72) Inventor: Braun, Marcel, 3510 Konolfingen (CH); Smets, Jacques, 1804 Corsier-sur Vevey (CH); Marie, Vanessa, 1700 Fribourg (CH)
(74) Representative: Marquardt, Ulf

(57) **Abstract**

The present invention relates generally to the prevention of the generation of off-flavours in food products. In particular is the present invention directed to the formulation of lipid raw material mixes and food products containing long chain polyunsaturated fatty acids, and to compositions and processes for stabilizing such products against the formation of off-flavours, such as fishy off-odours. One embodiment of the present invention is a composition comprising an LC-PUFA containing oil and lecithin, wherein weight ratio of lecithin to LC-PUFA is at least about 25 : 75.

## Description

The present invention relates generally to the prevention of the generation of off-flavours in food products. In particular is the present invention directed to the formulation of lipid raw material mixes and food products containing long chain polyunsaturated fatty acids, and to compositions and processes for stabilizing such products against the formation of off-flavours, such as fishy off-odours.

The importance of long chain polyunsaturated fatty acids (LC-PUFA) in the diet of humans, and particularly infants, is now well established (see e. g., WO 96/40106). Typical dietary sources of LC-PUFA are organ meats, fish, eggs and human breast milk. However, present day diets are frequently deficient in LC-PUFA, resulting in a need to supplement the diet with a source of LC-PUFA.

Sources of LC-PUFA supplements include egg yolk phospholipids, and triglyceride oils extracted from fish and marine microorganisms. The use of egg yolk phospholipids and/or marine triglyceride oils to supply LC-PUFA in infant formula is taught, e.g., in U. S. Patent No. 4,670,285 and in WO 96/10922. Use of microbial triglyceride oils to supply LC-PUFA in infant formula is taught in U. S. Patent Nos. 5,374,657, 5,397,591 and 5,550,156. Another source to supply LC-PUFA in infant formula is a lipid extract from human placenta, taught in European Patent No. 0 140 805.

Phospholipids (gums) are already added to foods (including infant formula), and these phospholipids are generally vegetable-derived phospholipids because they are relatively inexpensive. However, the levels of phospholipid added are generally less than 0.5% by weight of the fat blend, especially in infant formulas, and they are added to improve the physical properties of the product. For example, phospholipids may be added as emulsifiers or wetting agents.

Because of their degree of unsaturation, LC-PUFA are prone to oxidative degradation. Preserving the double bonds of the LC-PUFA through processing and storage is a critical issue in the preparation and distribution of infant formula, baby food and other nutritional supplements containing such materials.

European Patent No. 0 404 058 describes the addition of alpha-tocopherol and/or ascorbylpalmitate as antioxidants during preparation of LC-PUFA -containing mixtures to reduce oxidative degradation. The LC-PUFA - containing materials are added to a mixture containing antioxidants in amounts to give final concentration of from 150-300 ppm, and the mixture typically contains mono-and diglyceride emulsifiers. U. S. Patent No. 5,855,944 describes a process for stabilizing LC-PUFA containing marine oils by treating the oils with silica, steam deodorizing the oil, and then adding to the oil a mixture of food-grade lecithin, alpha-tocopherol, and ascorbyl-palmitate in a total amount of 1000-4000 ppm of the mixture.

WO 00/54575 and WO 97/35488 describe that the addition of up to 16% lecithin to the lipid mix have a stabilizing effect against oxidative degradation.

However up to now, no progress was made with respect to the reduction of the generation of fishy odours. The inhibition of the very disturbing fishy off-odours in the final food products, are of great importance to the consumer. The formation of ppb amounts of fishy off-flavour compounds, are sufficient to produce a strong fishy off-odour, even though the concentration of the precursor LC-PUFAs are not substantially reduced.

There remains a need for an inexpensive, effective method for inhibition of formation of fishy off flavours in lipid premixes and final products containing LC-PUFA.

Consequently, it was the object of the present invention to provide the art with a way to inhibit the formation of fishy off flavours in lipid premixes comprising LC-PUFA effectively and inexpensively.

This object is achieved by a composition in accordance with claim 1, a product in accordance with claim 17 and a use in accordance with claims 19 and 20.

The present inventors were surprised to find that the addition of lecithin to an LC-PUFA containing fat mixture in a weight/weight ratio of lecithin to LC-PUFA of at least about 25 : 75 achieves this object of the present invention.

Accordingly, one embodiment of the present invention is a composition comprising an LC-PUFA containing oil and lecithin, wherein weight ratio of lecithin to LC-PUFA is at least about 25 : 75. In preferred embodiments of the present invention the weight ratio of lecithin to LC-PUFA is at least about 30:70, 35:65, 40:60, 45:55, 50:50, 55:45, 60:40, 65:35, 70:30, 75:25, 80:20, 85:15, 90:10 or 95:5.

In a particular preferred embodiment is the weight ratio of lecithin to LC-PUFA at least about 55:45, even more preferred between about 60:40 - 95:5, most preferably between about 65:35 and 85:15.

The source of the LC-PUFA containing fat or oil is not critical to the present invention. Any such source that is known in the art can be employed. The skilled person generally knows sources of unsaturated fatty acids. Typical sources of DHA, for example, are fish oil or oils from micro organisms, such as Cryptecodinum cohnii. EP 0 515 460, for example, discloses a method for obtaining oil rich in DHA which is present in the biomass of cultivated dinoflagellates. WO 02/072742 discloses oils rich in DHA, ARA, DHGLA and EPA. A typical source of ARA, for example, is egg lecithin or biomass of fermentation processes (Mortierella alpina), the latter may be obtained according to the process disclosed in EP 0 568 608. If very pure preparations are desired it may be advantageous to prepare the LC-PUFA containing fat or oil synthetically. However, generally it is preferred that the LC-PUFA containing oil is selected from the group consisting of a marine oil, an oil produced by microorganisms, an oil produced by one-celled plants, an oil produced by multi-cellular plants or an oil of animal origin, or mixtures thereof.

The capability of lecithin to inhibit the formation of fish odour might be most beneficial when the LC-PUFA containing oil is fish oil.

The composition can be any composition where it is desired to avoid the generation of fishy odours. For example, the composition might be a medicament or a cream for topical application. Preferably, however, the composition is a food composition.

The composition of the present invention may be enriched in unsaturated fatty acids, preferably in polyunsaturated fatty acids. The PUFA (poly unsaturated fatty acids) may include omega-3 fatty acids and omega-6 fatty acids. Typical examples of PUFAs used in the present invention are docosahexaenoic acid (DHA), arachidonic acid (ARA), linoleic acid, alpha-linolenic acid, eicosapentaenoic acid and erucic acid.

In a particular preferred embodiment of the present invention the composition may be enriched in long chain poly unsaturated fatty acids. PUFAs are considered LC-PUFAs if the carbon chain comprises 18 C-atoms or more.

The composition of the present invention may be enriched in C18, C20 and/or C22 ω-6 polyunsaturated fatty acids. The weight ratio of omega-6 fatty acids and omega-3 fatty acids in the composition of the present invention is preferably between 1:2 to 8:1, more preferably between 4:1 to 8:1.

The polyunsaturated fatty acids may at least partially be present in form of free fatty acids. They may also at least partially be present in a mono-, di- and/or triglyceride form. This glyceridic form contributes to the stability of the PUFA and hence helps to avoid the generation of fishy odour.

It may further be advantageous if the composition of the present invention further comprises an antioxidant. The type of antioxidant is not critical, however, in case the composition is a food product or a medicament a food grade antioxidant is required. In cases where the composition is a product for topical administration a food grade antioxidant is at least strongly preferred. Antioxidants may help to avoid that LC-PUFAs are oxidized, which will lead to a loss of product value. Additionally, antioxidants can help to inhibit the formation of fishy odours. As antioxidant with particular beneficial properties in terms of avoiding fish odour generation extracts of citrus fruits, in particular lemons, can be mentioned. Further examples of suitable antioxidants may be selected from the group consisting of ascorbic acid; glutathione; lipoic acid; uric acid; carotenoids, e.g., lycopene, carotene; tocopherols; ubiquinone; hydrochinone; polyphenolic antioxidants, e.g., resveratrol, flavonoids; ascorbylpalmitate; galates; BHA; BHT; TBHQ; sulfites; retinols; carotenoides; flavonoids; tea extracts; rosemary extracts; nitrites; EDTA, citric acid, phytic acid; derivatives and/or mixtures thereof.

The amount of antioxidant that can be used in the framework of the present invention is not particularly limited and will depend on the type of antioxidant used. Those skilled in the art will be able to determine appropriate amounts. However, generally it is preferred if the antioxidant is added to the composition in an amount of about 0.001 weight-% to 1 % weight-%, preferably of about 0.01 weight-% to 0.5 weight-% with respect to the total composition.

The composition of the present invention may be a lipid raw material mix. In this case the long chain poly unsaturated fatty acids may represent about 1-75 weight-% of the composition, preferably about 3 - 50 weight-% of the composition, most preferred about 5-35 weight-% of the composition.

The composition may also further comprise a carbohydrate source, a protein source, and/or a further fat source.

A typical embodiment of, e.g., a lipid raw material mix, of the present invention is characterized in that after storage for 1 month at 40°C no development of a fishy odour is perceivable.

The present invention also relates to a product comprising a composition of the present invention.

Such a product may be a LC-PUFA containing raw material, in particular a marine oil, an oil produced by microorganisms, an oil produced by one-celled plants, an oil produced by multi-cellular plants or an oil of animal origin, a nutritional complete formula, a dairy product, a chilled or shelf stable beverage, a mineral or purified water, a liquid drink, a soup, a dietary supplement, a meal replacement, a nutritional bar, a confectionery, a milk or a fermented milk product, a yoghurt, a milk based powder, an enteral nutrition product, an infant formula, an infant nutritional product, a cereal product or a fermented cereal based product, an ice-cream, a chocolate, coffee, a culinary product such as mayonnaise, tomato puree or salad dressings, a health care product, a cosmetic product, a pharmaceutical product, or a pet food.

In case the product is not a raw material but a product ready for consumption it may often also further comprise a carbohydrate source, a protein source, and/or a further fat source.

The final product may comprise an amount of LC-PUFA that corresponds to the intended purpose of the product. A typical food composition may, however comprise, in percent by weight, 0.01-0.5%, preferably 0.015-0.4%, most preferably 0.02 -0.2%, for example 0.06% of LC-PUFAs.

If the product is a nutritional composition, it preferably comprises other constituents, such as macro- and micronutrients, functional food ingredients, for example. It may, for example, comprise further lipids. Typical lipid sources that may be used include milk fat, safflower oil, egg yolk lipid, canola oil, olive oil, coconut oil, palm oil, palm kernel oil, palm olein, soybean oil, sunflower oil. Medium chain triglycerides (MCT), which are defined herein as triglycerides comprising fatty acids with acyl chains of 6-12 carbon atoms (C6-C12) may also be included

Generally, fatty acids are preferably present in the form of triglycerides. They may, however, also be present in the form of free fatty acids, esters of other alcohols than glycerol or in the form of phospolipids.

In a final nutritional composition the lipids may provide 30-50%, preferably 35-45% of the total energy of the nutritional composition.

As protein or carbohydrate source, generally any protein source and/or carbohydrate source suitable as ingredients in nutritional compositions may be used.

The dietary protein, which may be used may be any suitable dietary protein; for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey protein are particularly preferred. The protein may be intact, hydrolysed protein, partially hydrolysed protein, free amino acids or a mixture of these. The protein source preferably provides from about 7 to 25% of the energy of the composition, more preferably 7-15%, most preferably 8-13%.

If the nutritional composition includes a carbohydrate source, any carbohydrates suitable for use in nutritional compositions may be used, for example digestible carbohydrates, such as maltodextrin, maltose, sucrose, lactose, glucose, fructose, corn syrup, corn syrup solids, rice syrup solids, starch, such as cereal starch, rice starch, corn starch, and mixtures thereof. The carbohydrate source preferably provides about 30% to about 70%, preferably 40-60% of the energy of the nutritional composition, for example if it is a complete nutritional composition.

Dietary fibre (non-digestible carbohydrates) may also be present in a nutritional composition according to the present invention if desired. Numerous types of dietary fibre are available. For example, oligosaccharides, such as fructo-oligo-saccharides, galactooligosaccharides, xylo-oligosaccharides, fuco-oligosaccharides, manno-oligosaccharides, just to mention a few, may be added.

The composition may comprise further ingredients, which are designed to meet the nutritional needs of the particular human being, or provide further benefits or functionalities. For example, the composition is preferably "nutritionally complete", that is it contains adequate nutrients to sustain healthy human life for extended periods. Preferably, the composition comprises vitamins and minerals. Also trace elements may be supplied.

If necessary, the product may contain emulsifiers and stabilisers such as citric acid esters of mono-and di-glycerides. The emulsifier may be selected from the group consisting of mono- and di-glycerides, acetic acid esters of mono/di-glycerides, lactic acid esters of mono/di-glycerides, diacetyl tartaric acid esters of mono/di-glycerides, succinic acid esters of mono glycerides, sorbitan esters, sucrose esters, polyglycerol esters, calcium stearoyl lactate and mixtures thereof.

The product may optionally contain other substances, which have a beneficial effect, such as lactoferrin, nucleotides and/or nucleosides.

A nutritional composition may further comprise a probiotic micro organism, preferably selected from the geni Bifidobacterium, Lactobacillus, Strepotococcus, and mixtures of these.

The present invention further relates to a method to inhibit the formation of fishy odours from an LC-PUFA containing oil composition comprising the steps of adding lecithin to the LC-PUFA containing oil composition in a weight/weight ratio of lecithin to LC-PUFA of at least about 25 : 75, or preferably in the weight ratios exemplified above.

Hence, one embodiment of the present invention is the use of the composition of the present invention to inhibit the formation of fishy odours from an LC-PUFA containing oil.

The present invention relates in general to the use of lecithin in compositions comprising an LC-PUFA containing oil to at least partially inhibit the formation of fishy odours.

Preferably, lecithin is used in a weight ratio of lecithin to LC-PUFA of at least about 30:70, 35:65, 40:60, 45:55, 50:50, 55:45, 60:40, 65:35, 70:30, 75:25, 80:20, 85:15, 90:10 or 95:5.

Lecithin is known to those of skill in the art.

Commercial lecithin may be a mixture of phospholipids in oil. The lecithin may be obtained by degumming the extracted oil of the seeds. The lecithin is a mixture of various phospholipids, and its composition depends on the origin of the lecithin. Any lecithin is well-suited for the present invention. Typical lecithin sources are known to those skilled in the art. Examples for major sources of lecithin are soybean oil, sunflower oil, and/or egg yolk. Also synthetically prepared lecithin can be used.

The main phospholipids in lecithin from soya and sunflower are phosphatidyl choline, phosphatidyl inositol, phosphatidyl ethanolamine and phosphatidic acid. They are often abbreviated to PC, PI, PE, and PA respectively.

To modify the performance of lecithin, i.e., to make it suitable for the product to which it is added, lecithin may be modified. One such modification is en enzymatic hydrolysation, e.g. by phospholipase A2, so that at least a part of the phospholipids have at least one fatty acid removed by the phospholipase.

The term "lecithin" within the disclosure of the present invention is meant to include such at least partially modified lecithins.

In particular satisfying results are achieved if lecithin is used in the composition in at least the same amount on a weight by weight basis as the LC-PUFA containing oil and preferably is the weight ratio of the used lecithin to LC-PUFA in the composition at least about 55:45, more preferable at least about 60:40 - 95:5, most preferably between 65:35 and 85:15.

In the use of the present invention lecithin can advantageously be used in combination with an antioxidant to further support the effect of lecithin against the development of fishy odour. Preferably, the antioxidant is selected from the group consisting of ascorbic acid; glutathione; lipoic acid; uric acid; carotenoids, e.g., lycopene, carotene; tocopherols; ubiquinone; hydrochinone; polyphenolic antioxidants, e.g., resveratrol, flavonoids; ascorbylpalmitate; galates; BHA; BHT; TBHQ; sulfites; retinols; carotenoides; flavonoids; tea extracts; rosemary extracts; nitrites; EDTA, citric acid, phytic acid; derivatives and/or mixtures thereof and/or is added to the composition in an amount of about 0.001 weight-% to 1 % weight-%, preferably about 0.01 weight-% to 0.5 weight-%.

As those skilled in the art will understand it is possible to freely combine any features of the present invention described in this specification without departing from the scope of the present invention as originally disclosed.

Further features and advantages of the present invention will be apparent from the following Examples and Figures.

Figure 1 shows the formation of selected volatile flavour compounds (1-penten-3-on, trans-trans-2,4-heptadienal and trans-2-pentenol), after a storage time of 1 month (40°C, ∼50% air head space).

Figure 2 shows the formation of 1-penten-3-on after a storage time of 1 month (40°C, ∼50% air head space) with an enlarged scale.

Figure 3 shows the perception of fishy odours in fish oil-lecithin mixes.

### Examples

Materials:
- Liquid soy bean lecithin, Topcithin NGM, Sugro Remis (Item: 001957) Lot 600665
- Fish oil NIF, ex Nestrade / Sofinol, Remis (Item: 000199) lot 601201

### Preparation of lecithin / fish oil mixes:

50g of the following weight-based mixtures were freshly prepared:
1) 99% fish oil + 1% lecithin
2) 98% fish oil + 2% lecithin
3) 95% fish oil + 5% lecithin
4) 90% fish oil + 10% lecithin
5) 85% fish oil + 15% lecithin
6) 80% fish oil + 20% lecithin
7) 70% fish oil + 30% lecithin
8) 60% fish oil + 40% lecithin
9) 50% fish oil + 50% lecithin
10) 40% fish oil + 60% lecithin
11) 30% fish oil + 70% lecithin
12) 20% fish oil + 80% lecithin
13) 100% fish oil
14) 100% lecithin
15) Ref. 100% fish oil (-25°C)
16) Ref. 100% lecithin (-25°C)
The mixtures were prepared in 100ml brown-glass Pyrex flasks with Teflon seal caps. In a 40°C water bath, the lipid samples were warmed up and carefully rotated/mixed manually to homogenize.

Storage: 1 Month at 40°C (oven), every 2-3 days samples were rotated/mixed for some seconds (except for the frozen samples)

### Sensory Test (technical olfactory test)

The olfactory test was performed with 7 experienced tasters. The samples were compared with the non-fishy reference (100% lecithin stored at -25°C / 1 month) and the very fishy reference (100% fish oil stored at 40°C / 1 month), in a comparative odour test with indication of fishy odour intensity on a graphic scale (0 = not fishy, 10 = very fishy).

### Analysis of volatile flavour compounds by SPME-GC/MS

The solid phase micro extraction (SPME) - gas chromatography (GC) / mass spectrometry (MS) method of analysis was carried out as follows:

### Example for analysis of flavour compounds

1.0 g of lipid sample is weighted into a 20ml crimp top vial and a magnetic stirring bar is added. 50 µl of internal standard ethylvalerate solution (45µl ethylvalerate in 100ml water) and 50 µl of internal standard 4-methyloctanoic acid solution (75µl of methyloctanoic acid in 250ml water) were added, followed by closing of the vial. The vial is then placed into a water bath at 65°C using a magnetic hot plate stirrer with contact thermometer at a speed range of about 870 rpm. After an equilibration time of 30min the fibre assembly (SPME Fiber Assemply, 2cm, 50/30µm DVB/CAR/PDMS StableFlex mounted in holder, both manufactured by SUPELCO Bellefonte, USA) is inserted by piercing the septa (depth gauge at 20mm) and by exposing the fibre completely to the headspace above the sample solution. After a sorption time of 30min the fibre is retracted and removed into the fibre assembly and taken out of the vial. The fibre assembly is immediately injected into the GC injector (depth gauge at 30mm) and the separation is started by exposure of the fibre at the same time. After 5min the fibre is retracted and removed from the injector. The flavour compounds are separated by gas chromatography on a FFAP capillary column (50m, 0.2mm inner diameter, 0.3 µm coating, Agilent Technologies USA) using helium as carrier gas and a temperature gradient from 40°C to 250°C. The separated compounds are detected and identified by mass spectrometry. Relative quantification is done by calculation of the response to flavour compounds in relation to the known amounts of the internal standards ethylvalerate (neutral compounds) and 4-methyloctanoic acid (acidic compounds). The same method was applied on the automated sample preparation system (Gerstel/Agilent) for SPME analysis (without magnet stirring).

### Results

### Analysis of volatile flavor compounds by SPME-GC/MS

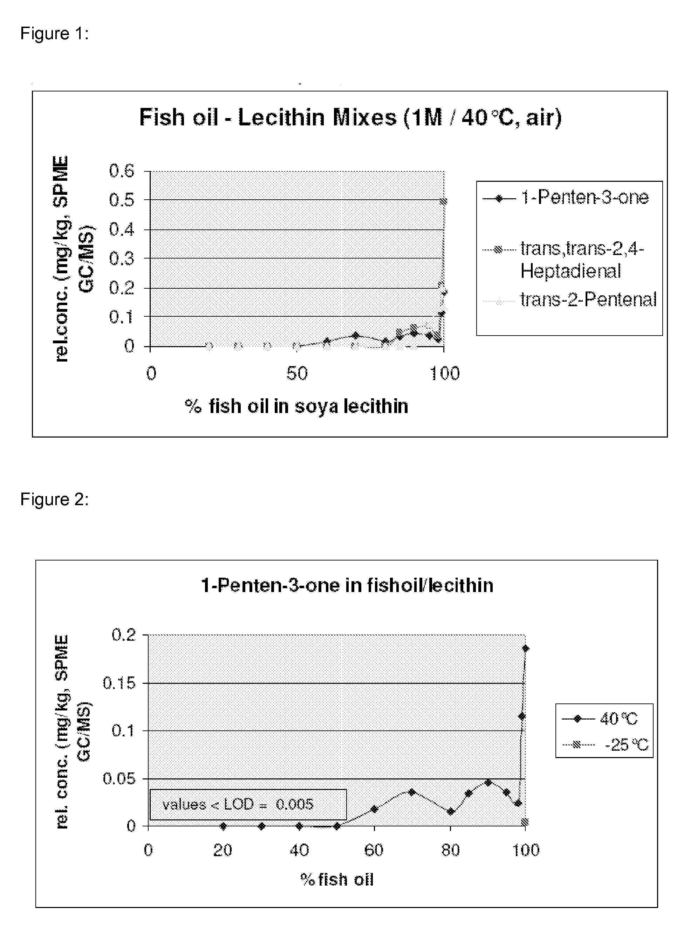

Figures 1 and 2 display graphs showing the formation of selected volatile flavor compounds, after a storage time of 1month (40°C, ∼50% air head space). No substantial formation of fish oil degradation products was detected by SPME-GC/MS analysis at higher dosage of lecithin (range 75 weight-% or less LC-PUFA in lecithin). At higher fish oil concentrations, e.g., the following selected odour compounds were formed
- 1-penten-3-one, strong fishy, threshold value (oil, nasal): 0.0007 mg/kg [4], LOD (limit of detection, SPME-GC/MS) ∼0.005 mg/kg.
- tr2, tr4-heptadienal, fatty, threshold value (oil, nasal): 10 mg/kg [4], LOD (limit of detection, SPME-GC/MS) ∼0.005 mg/kg.
- tr2-pentenal, pungent, apple, threshold value (oil, nasal): 2.3 mg/kg [4], LOD (limit of detection, SPME-GC/MS) ∼0.005 mg/kg.

Only the very fishy compound 1-penten-3-one was detected in concentrations above the threshold value in samples containing high concentrations of fish oil.

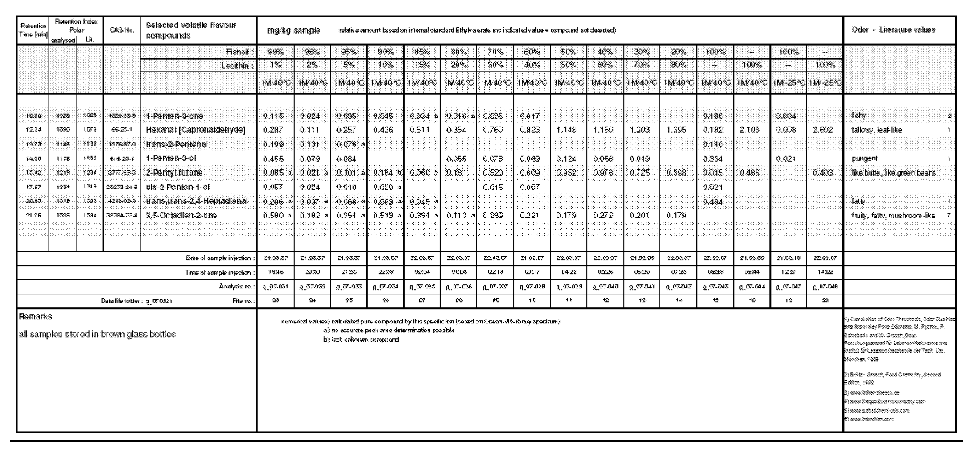

### Sensory Test (odour)

Within the concentration range from 0 - 40% fish oil in lecithin, no significant fishy odour was perceived, whereas for concentrations above 40% fish oil increasing fishy odour formation was detected.

The detection of fishy odour (threshold value (oil, nasal): 0.0007 mg/kg) fits quite well with the detection of the strong fishy compound 1-penten-3-one (limit of detection ∼0.005 mg/kg), even the sensitivity of the SPME-GC/MS method is not sufficient to analyze concentrations at threshold value.

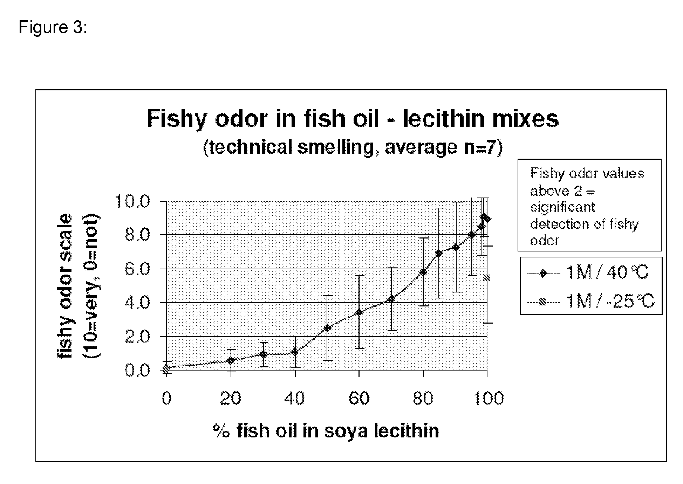

Figure 3 shows the results of the sensory test.

The efficiency of Soya lecithin addition in the inhibition of the formation of fishy odour from LC-PUFA containing samples was clearly shown. 1-penten-3-one was successfully applied for monitoring fishy off-taste formation in fish oil containing lipid samples. The fishy off-odour appears to be, however, not substantially masked by the soybean lecithin aroma.

Without wishing to be bound by theory the inventors presently assume that the hypothesis of mechanism on fishy odor inhibition is based on formation of Schiff bases due to a reaction of ketones and aldehydes (e.g. 1-penten-3-one) with the amino groups of phospholipids (e.g. phosphatidyl ethanolamine, phosphatidyl serine). The formed derivative is not volatile and, possibly for this reason no fishy off-odour is detectable.

These results were confirmed by a pilot plant trial showing no off-taste formation of the produced Lactogen powder even after storage for 33 month (20°C, gassed). The fish oil addition was done by lecithination (0.45% mix on powder) of a mixture of soy bean lecithin (Topcithin 200) / fish oil

The following table shows the results of this pilot plant trial:

| | **Odor / Taste** | **ARA 20:4 n-6** | **EPA 20:5 n-3** | **DHA 22:6 n-3** |
|---|---|---|---|---|
| | | **g FA / 100g prod.** | **g FA / 100g prod.** | **g FA / 100g prod.** |
| **Fish oil** (NIF, Lot 300943) | | | | |
| 1 month 4°C, air | normal | 1.53 | 7.00 | 24.54 |
| 1 month 40°C, air | fishy, oxidized | 2.45 | 6.94 | 23.95 |
| | | | | |
| **Fish oil / Lecithin 50 : 50** | | | | |
| 1 month 4°C, air | normal | | | |
| 1 month 40°C, air | normal, trace fishy | 0.77 | 3.60 | 12.22 |
| | | | | |
| **Fish oil / Lecithin 30 : 70** | | | | |
| 1 month 4°C, air | normal | | | |
| 1 month 40°C, air | normal | 0.46 | 2.11 | 7.20 |
| | | | | |
| **Trial Lactogen** MFD 22.01.2004, base powder (0.32% lecithin + 0.13% fish oil, = 0.032% DHA) | Reconstitution 13% | | | |
| 1 month 4°C, air | normal | 0.01 | 0.014 | 0.017 |
| 1 month 40°C, air | normal | 0.01 | 0.013 | 0.017 |
| | | | | |
| 33 months 20°C, gassed (RBe, VAM, COB, BR) | normal, no fishy taste | -- | -- | -- |

The table shows that if liquid soybean lecithin is added to fish oil in a ratio of at least 50:50 or even better at 30:70 fish oil / lecithin, no substantial dish odour generation could be observed in the lipid mixes and in infant formula, even though challenged by storage in air at 40°C for 1 month and 33 months at 20°C, gassed. Without wishing to be bound by theory, the inventors presently presume that lecithin is most probably chemically binding fishy off-flavour compounds (Maillard-type of reaction), as no substantial fishy taste was observed in the lecithin-enriched samples.

## Claims

1. Composition comprising an LC-PUFA containing oil and lecithin, wherein weight ratio of lecithin to LC-PUFA is at least about 25 : 75.

2. Composition in accordance with claim 1, wherein the LC-PUFA containing oil is selected from the group consisting of a marine oil, an oil produced by microorganisms, an oil produced by one-celled plants, an oil produced by multi-cellular plants or an oil of animal origin, or mixtures thereof.

3. Composition in accordance with one of claims 1-2, wherein the LC-PUFA containing oil is fish oil.

4. Composition in accordance with one of claims 1-3, wherein the weight ratio of lecithin to LC-PUFA is at least about 55:45.

5. Composition in accordance with one of claims 1-4 wherein the weight ratio of lecithin to LC-PUFA is between about 60:40 - 95:5, preferably between about 65:35 and 85:15.

6. Composition in accordance with one of claims 1-5, wherein the composition is a food composition.

7. Composition in accordance with one of claims 1-6 wherein the composition is enriched in poly unsaturated fatty acids.

8. Composition in accordance with one of claims 1-7 wherein the composition is enriched in long chain poly unsaturated fatty acids.

9. Composition in accordance with one of claims 1-8 wherein the composition is enriched in C18, C20 and/or C22 ω-6 polyunsaturated fatty acids.

10. Composition in accordance with claims 1-9 wherein the polyunsaturated fatty acids are at least partially present in mono-, di- and/or triglyceride form.

11. Composition in accordance with one of claims 1-10 further comprising a carbohydrate source, and a protein source.

12. Composition in accordance with one of claims 1-11 further comprising an antioxidant.

13. Composition in accordance with claim 12, wherein the antioxidant is selected from the group consisting of ascorbic acid; glutathione; lipoic acid; uric acid; carotenoids, e.g., lycopene, carotene; tocopherols; ubiquinone; hydrochinone; polyphenolic antioxidants, e.g., resveratrol, flavonoids; ascorbylpalmitate; galates; BHA; BHT; TBHQ; sulfites; retinols; carotenoides; flavonoids; tea extracts; rosemary extracts; nitrites; EDTA, citric acid, phytic acid; derivatives and/or mixtures thereof.

14. Composition in accordance with one of claims 12-13, wherein the antioxidant is added to the composition in an amount of about 0.001 weight-% to 1 % weight-%, preferably about 0.01 weight-% to 0.5 weight-%.

15. Composition in accordance with one of claims 1-14, wherein the long chain poly unsaturated fatty acids represent about 1-75 weight-% of the composition, preferably about 3 - 50 weight-% of the composition, most preferred about 5-35 weight-% of the composition.

16. Composition in accordance with one of claims 1-15, **characterized in that** after storage for 1 month at 40°C no development of a fishy odour is perceivable.

17. Product comprising a composition in accordance with one of claims 1-16.

18. Product in accordance with claim 17 wherein the product is a LC-PUFA containing raw material, in particular a marine oil, an oil produced by microorganisms, an oil produced by one-celled plants, an oil produced by multi-cellular plants or an oil of animal origin, a nutritional complete formula, a dairy product, a chilled or shelf stable beverage, a mineral or purified water, a liquid drink, a soup, a dietary supplement, a meal replacement, a nutritional bar, a confectionery, a milk or a fermented milk product, a yoghurt, a milk based powder, an enteral nutrition product, an infant formula, an infant nutritional product, a cereal product or a fermented cereal based product, an ice-cream, a chocolate, coffee, a culinary product such as mayonnaise, tomato puree or salad dressings, a health care product, a cosmetic product, a pharmaceutical product, or a pet food.

19. Use of a composition in accordance with claims 1-16 to inhibit the formation of fishy odours from an LC-PUFA containing oil.

20. Use of lecithin in compositions comprising an LC-PUFA containing oil to at least partially inhibit the formation of fishy odours.

21. Use in accordance with claim 20, wherein lecithin is used in the composition in at least the same amount on a weight by weight basis as LC-PUFA containing oil.

22. Use in accordance with one of claims 19-21 wherein the weight ratio of lecithin to LC-PUFA in the composition is at least about 55:45.

23. Use in accordance with one of claims 19-21 wherein the weight ratio of lecithin to LC-PUFA in the composition is at least about 60:40 - 95:5, preferably between 65:35 and 85:15.

24. Use in accordance with one of claims 19-23 wherein the lecithin is used in the composition in combination with an antioxidant.

25. Use in accordance with claim 24, wherein the antioxidant is selected from the group consisting of ascorbic acid; glutathione; lipoic acid; uric acid; carotenoids, e.g., lycopene, carotene; tocopherols; ubiquinone; hydrochinone; polyphenolic antioxidants, e.g., resveratrol, flavonoids; ascorbylpalmitate; galates; BHA; BHT; TBHQ; sulfites; retinols; carotenoides; flavonoids; tea extracts; rosemary extracts; nitrites; EDTA, citric acid, phytic acid; derivatives and/or mixtures thereof.

26. Use in accordance with one of claims 24-25, wherein the antioxidant is added to the composition in an amount of about 0.001 weight-% to 1 % weight-%, preferably about 0.01 weight-% to 0.5 weight-%.
